# EUROPÄISCHE PATENTANMELDUNG

(11) **EP 0 897 916 A2**
(43) Veröffentlichungstag der Anmeldung: **24.02.1999**
(21) Anmeldenummer: 98115043.6
(22) Anmeldetag: 11.08.1998
(51) Int. Cl.: C07D 249/00, C07C 49/825, C08K 5/3475, C08K 5/3492

(54) **Neue 2-(2'-Hydroxyphenyl)benzotriazole und 2-Hydroxybenzophenone als Lichtschutzmittel für polymeres Material**

(30) Priorität: 18.08.1997 DE 19735791
(71) Anmelder: Clariant GmbH, 65929 Frankfurt am Main (DE)
(72) Erfinder: Mehrer, Mathias, Dr., 86456 Gablingen (DE); Stährfeldt, Thomas, Dr., 86356 Neusäss (DE); Zäh, Matthias, Dr., 86368 Gersthofen (DE)

(57) **Zusammenfassung**

Die vorliegende Erfindung bezieht sich auf neue Stabilisatoren der allgemeinen Formel (I) 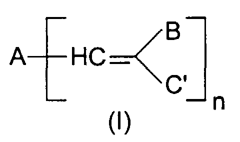 worin die Substituenten die in der Beschreibung definierte Bedeutung haben.

Die neuen Verbindungen sind ausgezeichnete Stabilisatoren für polymeres Material gegen den Einfluß von Licht und Wärme.

## Beschreibung

Die Erfindung betrifft neue extraktionsbeständige UV-Absorber auf Basis von 2-(2'-Hydroxyphenyl)benztriazolen und 2-Hydroxybenzophenonen, deren Verwendung zur Stabilisierung von polymerem Material, entsprechend stabilisierte Zusammensetzungen und ein Verfahren zur Stabilisierung organischer Polymere mit Hilfe dieser Verbindungen.

2-(2'-Hydroxyphenyl)benztriazole und 2-Hydroxybenzophenone werden seit geraumer Zeit zur Stabilisierung von organischem Material eingesetzt. Hierbei bereitet besonders die hohe Flüchtigkeit dieser Substanzklassen aus dem polymeren Material sowie auch die Extrahierbarkeit dieser Verbindungen in Kontakt mit extrahierenden Medien erhebliche Probleme. Es wurden deshalb bis heute zahlreiche Versuche unternommen, die negativen Begleiterscheinungen dieser Substanzklassen durch Vergrößerung des Molgewichts (z. B in EP-A-593936; EP-A-191582; EP-A-669330) und/oder durch eine feste Fixierung des UV-Absorbers an die polymere Matrix (US 4,652,656; DE-A-4438575) zu reduzieren. Dies führt jedoch nur in begrenztem Maße zum gewünschten Erfolg und es besteht nach wie vor ein großes Interesse an neuen Stabilisatoren mit verbessertem Eigenschaftsprofil.

Aus der Chemie der HALS-Stabilisatoren ist ein Prinzip bekannt (DE-A-3412227; DE-A-4327297; deutsche Patentanmeldung Nr. 19618197.6), welches eine schnelle Migration niedermolekularer Stabilisatoren an die Polymeroberfläche sowie eine Veränderung des Molgewichts des Stabilisators durch eine durch das Sonnenlicht hervorgerufene photochemische Reaktion beinhaltet. Durch diese Molgewichtsveränderung ändern sich sowohl das Migrations - als auch das Extraktionsverhalten des primär eingesetzten Stabilisators.

Aufgabe der vorliegenden Anmeldung war es, neue UV-Absorber auf Basis von 2-(2'-Hydroxyphenyl)benztriazolen und 2-Hydroxybenzophenonen zu entwickeln, welche zusätzlich eine photochemisch aktive Einheit im Molekül tragen und welche durch diese Einheit in der Lage sind, die geschilderte Funktion im obigen Sinne zu erfüllen.

Überraschenderweise wurde gefunden, daß UV-Absorber, die mit einer photochemisch aktiven Einheit auf Basis von Zimtsäure oder deren Derivaten kombiniert werden, die oben gestellten Anforderungen in hervorragender Weise gewährleisten.

Gegenstand der Erfindung sind deshalb Verbindungen der allgemeinen Formel (I), 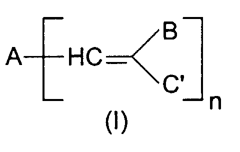 worin
n 1, 2 oder 3 ist und für die Bindungszahl von A steht
A für n = 1 einen ein- oder mehrfach durch C₁-C₄-Alkyl, vorzugsweise C₁-C₂-Alkyl, insbesondere Methyl; Trifluormethyl, C₁-C₄-Alkoxy, vorzugsweise C₁-C₂-Alkoxy, insbesondere Methoxy; Halogen, Cyan, Carboxy, Nitro, Amino, C₁-C₄-Alkylamino, vorzugsweise C₁-C₂-Alkylamino, insbesondere Methylamino; C₁-C₄-Dialkylamino, vorzugsweise C₁-C₂-Dialkylamino, insbesondere Dimethylamino; Acyl oder Acyloxy- substituierten aromatischen oder heteroaromatischen Rest mit 6 bis 20, vorzugsweise 6 bis 10, insbesondere 6 bis 8 C-Atomen im Ringgerüst bedeutet, wobei als Heteroaromaten beispielsweise Pyridin, Furan, Thiophen, Pyrimidin, Pyrrol, Imidazol, Pyrazol in Frage kommen, und auch eine ein- oder beidseitige Benzanellierung des aromatischen Rests möglich ist;
A für n = 2 einen bivalenten aromatischen Rest mit 6 bis 20 C-Atomen im Ringgerüst bedeutet, der entweder selbst bivalent ist, wie z.B. in den Formeln (II) bis (XI) 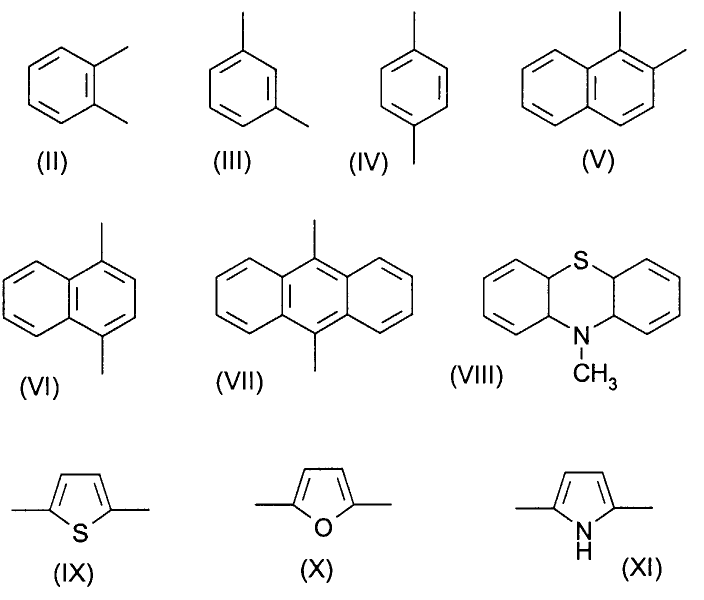 oder in dem zwei Reste durch eine Brücke verknüpft sind, wie z. B. in den Formeln (XII) bis (XVIII), 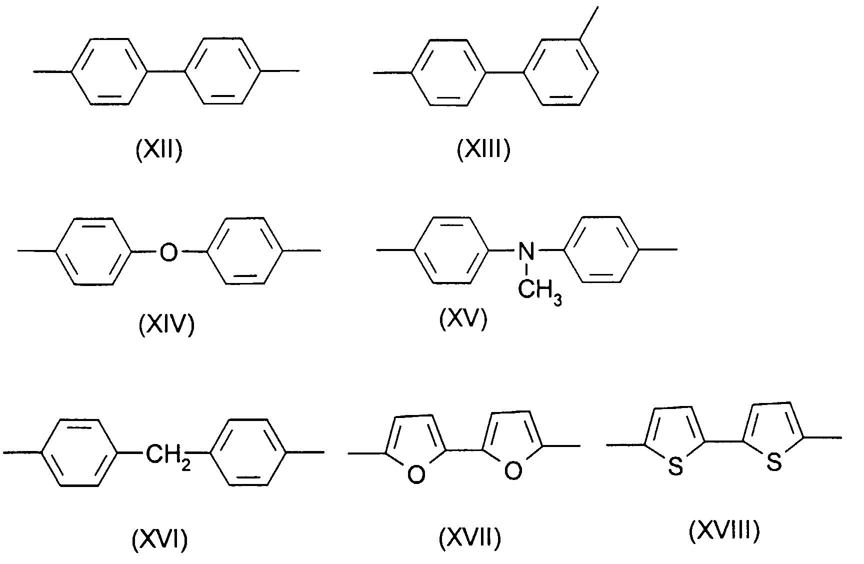 wobei diese Reste zusätzlich noch ein- oder mehrfach durch C₁-C₄-Alkyl, vorzugsweise C₁-C₂-Alkyl, insbesondere Methyl; Trifluormethyl, C₁-C₄-Alkoxy, vorzugsweise C₁-C₂-Alkoxy, insbesondere Methoxy; Halogen, Cyan, Carboxy, Nitro, Amino, C₁-C₄-Alkylamino, vorzugsweise C₁-C₂-Alkylamino, insbesondere Methylamino; C₁-C₄-Dialkylamino, vorzugsweise C₁-C₂-Dialkylamino, insbesondere Dimethylamino; Acyl oder Acyloxy substituiert sein können;
A für n = 3 einen trivalenten aromatischen Rest von 6 bis 20 C-Atomen z. B. der Formeln (XIX) bis (XXIII) bedeutet, 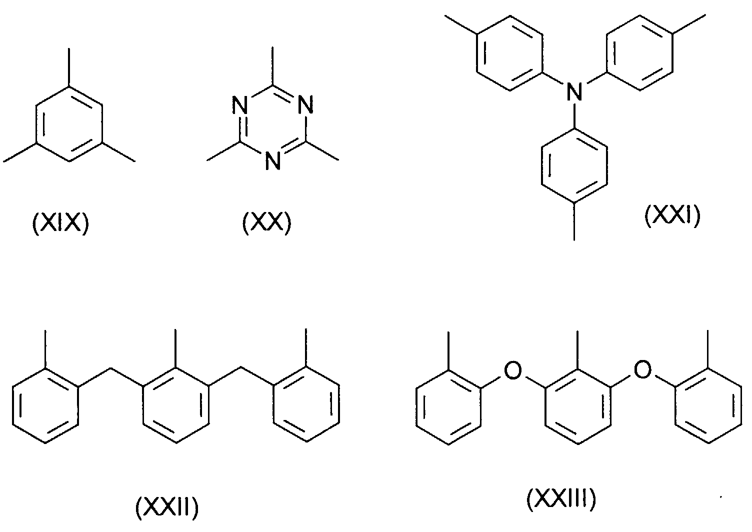 wobei dieser zusätzlich noch ein- oder mehrfach durch C₁-C₄-Alkyl, vorzugsweise C₁-C₂-Alkyl, insbesondere Methyl; Trifluormethyl, C₁-C₄-Alkoxy, vorzugsweise C₁-C₂-Alkoxy, insbesondere Methoxy; Halogen, Cyan, Carboxy, Nitro, Amino, C₁-C₄-Alkylamino, vorzugsweise C₁-C₂-Alkylamino, insbesondere Methylamino; C₁-C₄-Dialkylamino, vorzugsweise C₁-C₂-Dialkylamino, insbesondere Dimethylamino; Acyl oder Acyloxy substituiert sein kann;
B -C(=O)-D-E bedeutet
C' Wasserstoff, Cyan oder -C(=O)-D-E bedeutet, wobei
D gleichbedeutend mit -O- oder -NH- ist und
E für D = -O- einen Rest der Formel (XXIV) oder (XXV) bedeutet und 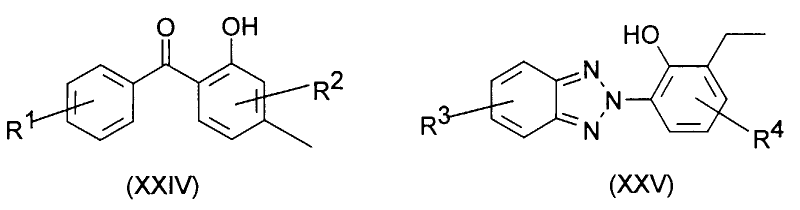
E für D = -NH- ein Rest der Formel (XXV) ist,
   wobei R¹, R², R³ und R⁴ einen oder mehrere Substituenten C₁-C₁₈-Alkyl, C₁-C₁₈-Alkoxy, Halogen, oder Cyan darstellen.

Als besonders geeignet erweisen sich Stabilisatoren, worin R¹, R² und R³ Halogen, C₁-C₁₂-Alkyl, C₁-C₁₂-Alkoxy oder Cyan und R⁴ C₁-C₁₂-Alkyl, C₁-C₁₂-Alkoxy oder Chlor bedeuten.

Ganz besonders gute Stabilisatoren sind Verbindungen, worin
- n: 1 ist,
- A: einen ein- oder mehrfach durch Methyl, Methoxy, Nitro oder Chlor substituierten Phenylrest bedeutet,
- B: -C(=O)-D-E bedeutet,
- C': Wasserstoff oder -C(=O)-D-E,
- D: -NH oder -O- ist,
- E: für D=-NH ein Rest der Formel (XXV) und für D=-O- ein Rest der Formel (XXIV) ist,
wobei
R¹ bis R⁴ unabhängig voneinander einen oder mehrere Methylreste darstellen.

Die Erfindung bezieht sich auch auf das allgemeine Herstellverfahren von Verbindungen der Formel (I) sowie deren Verwendung zur Stabilisierung von organischem Material, insbesondere von Kunststoffen, Anstrichmitteln, Ölen und Lacken.

Die Herstellung von Verbindungen der allgemeinen Formel (I) erfolgt durch Umsetzung von Verbindungen der allgemeinen Formeln (XXVI) oder (XXVII), 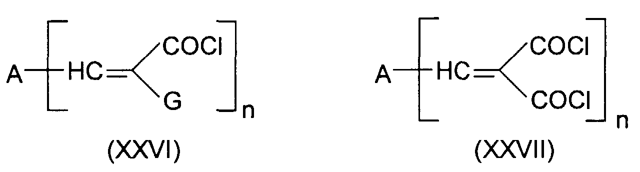 wobei G dann die Bedeutung von Wasserstoff oder Cyan hat, mit Verbindungen der allgemeinen Formel (XXVIII)

E―D―H (XXVIII)

wobei D und E die obigen Bedeutungen besitzen und es sich im Falle von D = -NH- auch um das Hydrochlorid (resp. das Salz) der eingesetzten Verbindung handeln kann. Solche Verbindungen können z. B. analog einer Vorschrift aus US 3,629,192 hergestellt werden. Die Verbindungen der Formeln (XXVI) und (XXVII) (Säurechloride) können auf literaturbekannten Wegen hergestellt werden, z. B. analog der in C. Ferri, Reaktionen der Organischen Synthese, Georg Thieme Verlag Stuttgart, 1978 oder anderen in konventionellen Lehrbüchern beschriebenen Methoden.

Die Herstellung von Verbindungen der allgemeinen Formel (I) erfolgt in polaren oder unpolaren, aprotischen Lösemitteln, besonders aber hochsiedenden aromatischen oder aliphatischen Kohlenwasserstoffen wie z. B. Ligroin, hochsiedenden Petrolether, Toluol, Xylol, Mesitylen oder Decalin. Die Reaktionstemperatur kann zwischen Raumtemperatur und Rückflußtemperatur des entsprechenden Lösemittels gewählt werden. Die Reaktionsdauer beträgt in der Regel zwischen 2 und 24 h. Die Reaktion wird bevorzugt unter einer Inertgasatmosphäre (Stickstoff oder Argon) durchgeführt, wobei die Edukte der Formeln (XXVI) oder (XXVII) und der Formel (XXVIII) bevorzugt äquimolar oder Verbindungen der Formel (XXVI) oder (XXVII) im leichten Überschuß (5 bis 20 Mol-%) bezogen auf Verbindungen der Formel (XXVIII) eingesetzt werden. Die Abspaltung des entstehenden Chlorwasserstoffs kann durch Hilfsbasen, bevorzugt sekundäre oder tertiäre Amine wie z. B. Pyridin, Triethylamin, Piperidin oder Morpholin; insbesondere aber Triethylamin, herbeigeführt werden. Nach der Reaktion wird das Reaktionsgemisch aufgearbeitet, indem der unter Umständen vorhandene Überschuß an Verbindungen der Formel (XXVI) oder (XXVII) über eine Natronlauge-Wäsche entfernt wird, das Reaktionsprodukt aufgearbeitet und durch Kristallisation aus geeigneten Lösemitteln isoliert wird. Ein alternatives Herstellverfahren zur Herstellung von Verbindungen der allgemeinen Formel (I) besteht in der Umsetzung von Verbindungen der allgemeinen Formel (XXIX)

A⁅CHO]ₙ (XXIX)

mit Verbindungen der allgemeinen Formeln (XXX) oder (XXXI), 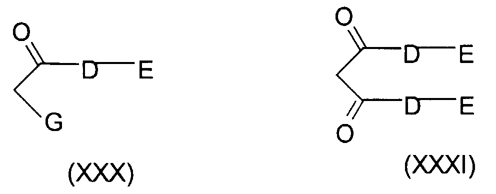 in denen die Symbole D, E und G die obengenannten Bedeutungen aufweisen. Die Synthese von Verbindungen der Formeln (XXX) oder (XXXI) wird z. B. in CA 2,099,154; US-4812498; US-4948666 oder US-4681905 beschrieben.

Für die Herstellung von Verbindungen der Formel (I) stehen typische Kondensationsreaktionen zur Verfügung, wie sie z. B. in C. Ferri, Reaktionen der Organischen Synthese, Georg Thieme Verlag Stuttgart, 1978 oder anderen konventionellen Lehrbüchern aufgeführt sind. Als Methode der Wahl hat sich hierbei die Kondensation in einem hochsiedenden organischen Lösemittel, z. B. Toluol, Xylol oder Mesitylen herausgestellt; die Reaktionstemperatur ist dabei zwischen 20 °C und der Siedetemperatur des Lösemittels zu wählen. Als Hilfsbase eignen sich hierbei aus der Fachliteratur bekannte Verbindungen, insbesondere z. B. Pyridin, Triethylamin, Piperidin oder Morpholin; bevorzugt ist die Verwendung von Piperidin, Pyridin oder deren Salze mit Essigsäure. Das sich abscheidende Reaktionswasser wird über einen Wasserabscheider dem Reaktionsgemisch kontinuierlich entzogen. Die Verbindungen der Formel (I) lassen sich nach Ablauf der Reaktion aus geeigneten Lösemitteln umkristallisieren.

Die erfindungsgemäßen Verbindungen eignen sich in hervorragender Weise zum Stabilisieren von organischem Material gegen die Einwirkung von Licht, Sauerstoff und Wärme. Sie werden dem zu stabilisierenden Material in einer Konzentration von 0.001 bis 5 Gew.-%, vorzugsweise von 0.02 bis 1 Gew.-%, bezogen auf das organische Material, vor, während oder nach seiner Herstellung, entweder alleine oder in Kombination mit weiteren Additiven zugesetzt. Unter organischem Material sind beispielsweise Vorprodukte für Kunststoffe, Lacke, Anstrichmittel und Öle, insbesondere jedoch Kunststoffe, Anstrichmittel, Lacke und Öle selbst, zu verstehen. Gegenstand der vorliegenden Erfindung ist außerdem ein gegen die Einwirkung von Licht, Sauerstoff und Wärme stabilisiertes organisches Material, insbesondere Kunststoffe, Anstrichmittel, Lacke und Öle, welche Verbindungen der Formel (I) in den oben angegebenen Konzentrationen enthalten. Zu diesen Materialien gehören beispielsweise Stoffe, wie sie in der deutschen Patentanmeldung Nr. 19719944.5 auf den Seiten 44 bis 50 beschrieben sind, worauf hier ausdrücklich Bezug genommen wird.

Das durch die erfindungsgemäßen Verbindungen der Formel (I) stabilisierte organische Material kann gegebenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe. Antioxidantien und Lichtstabilisatoren, die neben den erfindungsgemäßen Verbindungen zugesetzt werden, sind beispielsweise Verbindungen auf der Basis sterisch gehinderter Amine oder sterisch gehinderter Phenole oder Schwefel oder Phosphor enthaltende Costabilisatoren. Als geeignete zusätzliche in Kombination einsetzbare Additive sind beispielsweise Verbindungen verwendbar, wie sie in der deutschen Patentanmeldung Nr. 19719944.5 auf den Seiten 51 bis 65 beschrieben sind, worauf hier ausdrücklich Bezug genommen wird.

Die Additive der allgemeinen Formel (I) werden nach den allgemein üblichen Methoden in die organischen Polymere eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglicher Verdunstung des Lösemittels, kann die Einarbeitung erfolgen. Die Verbindungen sind auch wirksam, wenn sie in ein bereits granuliertes Polymer nachträglich in einem gesonderten Verarbeitungsschritt eingebracht werden. Die erfindungsgemäßen Verbindungen der Formel (I) können auch in Form eines Masterbatches, der diese Verbindungen beispielsweise in einer Konzentration von 1 bis 75, vorzugsweise 2,5 bis 30 % enthält, den zu stabilisierenden Polymeren zugesetzt werden.

Die nachfolgenden Beispiele sollen die Erfindung näher beschreiben, ohne diese in irgendeiner Form einzuschränken. Alle erfindungsgemäßen Verbindungen werden durch Angabe ihres Schmelzpunktes sowie ihrer C,H,N,O-Elementaranalysen charakterisiert. Die Verbindungen wurden außerdem anhand ihrer ¹H- und ¹³C- NMR-Spektren eindeutig identifiziert.

### Beispiel 1: Herstellung der Verbindung 1

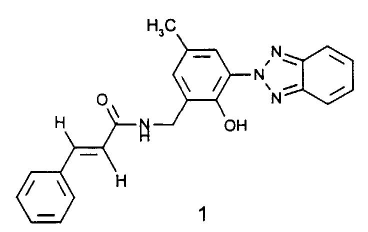

In einem Vierhalskolben mit Rückflußkühler, Tropftrichter, Innenthermometer und UPG-Rührer mit Stickstoff-Inertgasleitung werden 11,6 g (0,04 mol) 2-(2'-Hydroxy-3'-aminomethyl-5'-methylphenyl)-benztriazol-hydrochlorid in 100 ml wasserfreiem Toluol vorgelegt und in diese Suspension wird bei 20 °C 7,3 g (0,044 mol) frisch hergestelltes Zimtsäurechlorid (in 10 ml Toluol gelöst) getropft. Nach Zutropfende werden noch 10,1 g (0,10 mol) Triethylamin zugegeben, wobei eine exotherme Reaktion und Erwärmung der Reaktionsmischung auf ca. 40 °C beobachtet wird. Nach Abklingen der Reaktion wird noch weitere 6 h bei 50 °C gerührt und danach der Reaktionsansatz zur Trockene eingeengt. Das Rohprodukt wird in 200 ml Methylenchlorid aufgenommem, mit 200 ml 1 n Natronlauge versetzt, extrahiert, die Phasen getrennt und die organische Phase noch zweimal mit je 100 ml Wasser extrahiert. Die organische Phase wird über Magnesiumsulfat getrocknet, vom Trockenmittel filtriert und das Lösemittel abdestilliert. Der Rückstand wird aus 200 ml 1,4-Dioxan umkristalliert. Der Schmelzpunkt der blaßgelben Kristalle (13.6 g; 88.5%) beträgt 231°C.

| Elementaranalyse: | | | | | |
|---|---|---|---|---|---|
| C₂₃H₂₀N₄O₂ | Berechnet | %C 71.88 | %H 5.21 | %N 14.58 | %O 8.33 |
| (384.0) | Gefunden | %C 70.44 | %H 5.43 | %N 13.17 | %O 11.11 |

### Beispiel 2: Herstellung der Verbindung 2:

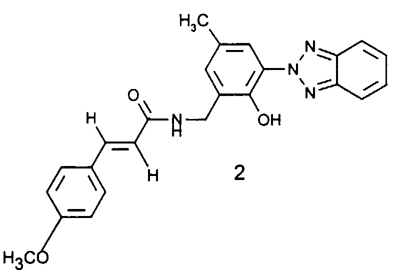

Analog Beispiel 1 aus 11.6 g (0.04 mol) 2-(2'Hydroxy-3'-aminomethyl-5'methylphenyl)-benztriazolhydrochlorid, 8.6 g (0.044 mol) p-Methoxyzimtsäurechlorid und 10.1 g (0.10 mol) Triethylamin in insgesamt 110 ml wasserfreiem Toluol. Nach Aufarbeitung und Verrühren des Rückstandes in Methanol, Filtration und Trocknen verbleiben 9.7 g (57.9%) eines hellen Pulvers vom Schmelzpunkt 215 °C.

| Elementaranalyse: | | | | | |
|---|---|---|---|---|---|
| C₂₄H₂₂N₄O₃ | Berechnet | %C 69.56 | %H 5.31 | %N 13.52 | %O 11.59 |
| (414.0) | Gefunden | %C 69.48 | %H 5.21 | %N 12.74 | %O 12.72 |

### Beispiel 3: Herstellung der Verbindung 3:

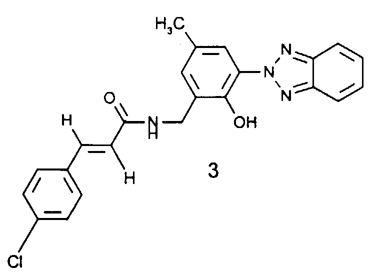

Analog Beispiel 1 aus 11,6 g (0,04 mol) 2-(2'Hydroxy-3'-aminomethyl-5'-methylphenyl)-benztriazolhydrochlorid, 8,8 g (0,044 mol) p-Chlorzimtsäurechlorid und 10,1 g (0,10 mol) Triethylamin in insgesamt 110 ml Toluol. Das Produkt wird nach der Aufarbeitung aus 200 ml Toluol umkristallisiert. Es verbleibt ein helles Pulver (9,7g; 57,9%) vom Schmelzpunkt 214°C.

| Elementaranalyse: | | | | | |
|---|---|---|---|---|---|
| C₂₃H₁₉ClN₄O₂ | Berechnet | %C 65.95 | %H 4.54 | %N 13.38 | %Cl 8.48 |
| (418.5) | Gefunden | %C 65.41 | %H 4.55 | %N 10.96 | %Cl 10.30 |

### Beispiel 4: Herstellung der Verbindung 4:

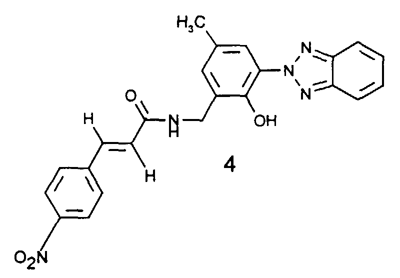

Analog Beispiel 1 aus 11,6 g (0,04 mol) 2-(2'Hydroxy-3'-aminomethyl-5'methylphenyl)-benztriazolhydrochlorid, 9,3 g (0,044 mol) 4-Nitrozimtsäurechlorid und 10,1 g (0,10 mol) Triethylamin in insgesamt 110 ml wasserfreiem Toluol. Nach Aufarbeitung und Kristallisation aus 100 ml Dimethylformamid verbleiben 6,9 g (40,2%) eines gelben Kristallpulvers vom Schmezpunkt 252 - 253 °C.

| Elementaranalyse: | | | | | |
|---|---|---|---|---|---|
| C₂₃H₁₉N₅O₄ | Berechnet | %C 64.33 | %H 4.43 | %N 16.32 | %O 14.92 |
| (429.0) | Gefunden | %C 63.48 | %H 4.37 | %N 15.90 | %O 16.03 |

### Beispiel 5: Herstellung der Verbindung 5

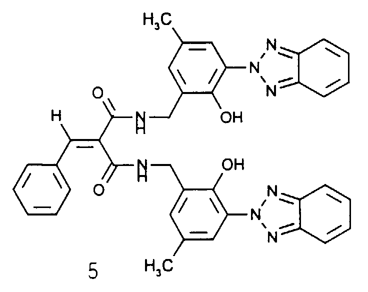

10,2 g (0,04 mol) 2-(2'-Hydroxy-3'-aminomethyl-5'-methylphenyl)-benztriazol-hydrochlorid werden in 50 ml wasserfreiem Toluol vorgelegt und hierzu eine frisch bereitete Lösung von 4,6 g (0,02 mol) Benzilidenmalonsäuredichlorid in 20 ml Toluol bei 20 °C zugetropft. Zu der gelben Suspension werden bei 20 °C 10,1 g (0,10 mol) Triethylamin zugegeben und die Suspension nach Abklingen der exothermen Reaktion noch weitere 4 h bei 50 °C gehalten. Das Lösemittel wird abdestilliert, der Rückstand in Methylenchlorid aufgenommen, die organische Phase mit 1 n NaOH ausgeschüttelt und schließlich noch zweimal mit je 100 ml Wasser extrahiert. Die organische Phase wird über Magnesiumsulfat getrocknet, vom Trockenmittel filtriert, das Lösemittel abdestilliert und der Rückstand aus 150 ml Xylol umkristallisiert. Es verbleiben 5,7 g (42,9%) eines beigen Pulvers vom Schmelzpunkt 232 °C.

| Elementaranalyse: | | | | |
|---|---|---|---|---|
| C₃₈H₃₂N₈O₄ | Berechnet | %C 68.67 | %H 4.82 | %N 16.87 |
| (664.0) | Gefunden | %C 68.75 | %H 5.13 | %N 16.75 |

### Beispiel 6: Herstellung der Verbindung 6

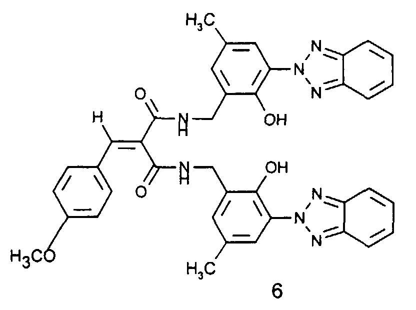

Analog Beispiel 5 aus 11,6 g (0,04 mol) 2-(2'Hydroxy-3'-aminomethyl-5'methylphenyl)-benztriazolhydrochlorid, 5,2 g (0,02 mol) p-Methoxybenzilidenmalonsäuredichlorid und 10,1 g (0,10 mol) Triethylamin in insgesamt 110 ml wasserfreiem Toluol. Der Rückstand wird nach der Aufarbeitung aus 50 ml Dimethylformamid umkristallisiert. Der Schmelzpunkt des gelben Pulvers (1,3 g; 9,4%) beträgt 219 - 225 °C.

| Elementaranalyse: | | | | | |
|---|---|---|---|---|---|
| C₃₉H₃₄N₈O₅ | Berechnet | %C 67.44 | %H 4.90 | %N 16.14 | %O 11.53 |
| (694.0) | Gefunden | %C 67.13 | %H 5.03 | %N 16.03 | %O 11.87 |

### Beispiel 7: Herstellung der Verbindung 7

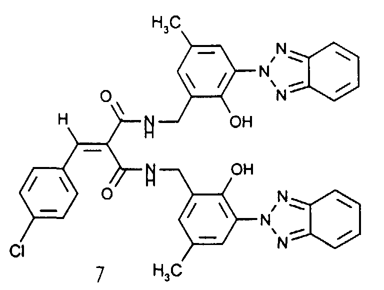

Analog Beispiel 5 aus 11,6 g (0,04 mol) 2-(2'Hydroxy-3'-aminomethyl-5'methylphenyl)-benztriazolhydrochlorid, 5,3 g (0,02 mol) p-Chlorbenzilidenmalonsäuredichlorid und 10,1 g (0,10 mol) Triethylamin in insgesamt 110 ml wasserfreiem Toluol. Der Rückstand wird nach der Aufarbeitung in 150 ml Tetrahydrofuran verrührt, abgesaugt und nachgetrocknet. Der Schmelzpunkt des beigen Pulvers (5,0 g; 35,8%) beträgt 207 - 210 °C.

| Elementaranalyse: | | | | |
|---|---|---|---|---|
| C₃₈H₃₁ClN₈O₄ | Berechnet | %C 64.11 | %H 5.09 | %N 16.12 |
| (698.5) | Gefunden | %C 65.28 | %H 4.44 | %N 16.03 |

### Beispiel 8: Herstellung der Verbindung 8:

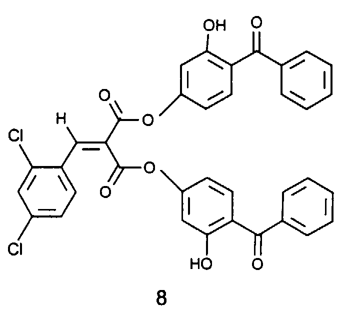

In einem Vierhalskolben mit Rückflußkühler, Tropftrichter, Innenthermometer, UPG-Rührer und Stickstoff-Inertgasleitung werden 14,0 g (0,047 mol) 2,4-Dichlorbenzilidenmalonsäuredichlorid, 20,1 g (0,094 mol) 2,4-Dihydroxybenzophenon und 10,1 g (0,1 mol) Triethylamin in 150 ml wasserfreiem Toluol zusammengegeben und 10 h unter Rückfluß zum Sieden erhitzt. Nach der Reaktion wird der Reaktionsansatz mit Wasser versetzt, die Phasen getrennt, die wäßrige Phase noch einmal mit 100 ml Toluol extrahiert und die vereinigten organischen Phasen über Natriumsulfat getrocknet. Nach dem Abdestillieren des Lösemittels wird der Rückstand mit Diisopropylether verrührt, der entstandene Festkörper filtriert und getrocknet. Der Schmelzpunkt des beigen Pulvers (20,2g; 65,8 %) beträgt 127 °C.

| Elementaranalyse: | | | | |
|---|---|---|---|---|
| C₃₈H₂₂Cl₂O₈ | Berechnet | %C 66.2 | %H 3.4 | %Cl 10.9 |
| (653.5) | Gefunden | %C 65.4 | %H 3.7 | %Cl 11.9 |

### Beispiel 9: Herstellung der Verbindung 9:

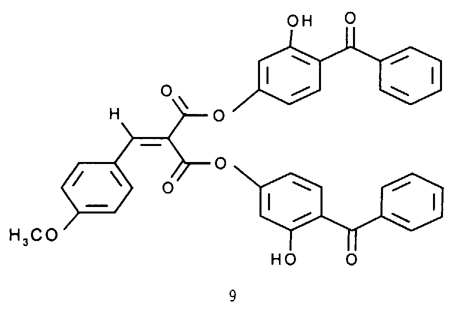

Analog Beispiel 8 aus 7,8 g (0,03 mol) 4-Methoxybenzilidenmalonsäuredichlorid, 12,8 g (0,06 mol) 2,4-Dihydroxybenzophenon und 6,7 g (0,066 mol) Triethylamin in 150 ml wasserfreiem Toluol. Der Rückstand wird nach Aufarbeitung an Kieselgel mit dem Laufmittelgemisch Petrolether/Essigester (1 : 1) chromatographiert. Es werden 11,9 g (64,6%) eines gelben, kristallinen Pulvers vom Schmelzpunkt 89 °C isoliert.

| Elementaranalyse: | | | |
|---|---|---|---|
| C₃₇H₂₆O₉ | Berechnet | %C 72.4 | %H 4.3 |
| (614.0) | Gefunden | %C 71.4 | %H 4.4 |

### Beispiel 10: Lichtstabilisierende Wirkung in Polypropylenspritzplatten

100 Gewichtsteile unstabilisiertes Polypropylen (®Hostalen PPK) werden zusammen mit 0.1 Gewichtsteilen Calciumstearat (Fa. Greven), 0.05 Gewichtsteilen Pentaerythrityl-tetrakis-3-(3,5-di-t-butyl-4-hydroxyphenyl)-propionat (®Hostanox O 10) und 1,0 Gewichtsteilen des zu prüfenden Stabilisators in einem Pulvermischer intensiv homogenisiert. Das so hergestellte Gemisch wird einmal mit einem Leistritz-Doppelschneckenextruder granuliert (gegenläufige Schneckenführung, Heizzonen 210 °C, 220 °C, 230 °C, 240 °C). Anschließend wird das Granulat zu Spritzplatten der Dicke 1 mm verarbeitet (Toshiba Doppelschneckenextruder, Heizzonen 210 °C, 220 °C, 230 °C, 240 °C). Die so hergestellten Spritzplatten werden in einem Schnellbewitterungsgerät (®Xenotest 1200) 358 h belichtet. Als Kriterium der Stabilität der Spritzplatten wurde die im Mikroskop erkennbare Verfärbung herangezogen (vgl. Tab. 1)

**Tab. 1**

| Verfärbung von 1 mm PP-Spritzplatten nach 358 h Belichtung (®Xenotest 1200) | |
|---|---|
| Zusätzlich zur Grundstabilisierung eingesetzter Stabilisator | Verfärbung |
| Keiner | Leichte Verfärbung sichtbar |
| Verbindung aus Beispiel 3 | Keine Verfärbung sichtbar |
| Verbindung aus Beispiel 9 | Keine Verfärbung sichtbar |

### Beispiel 11: Lichtstabilisierende Wirkung in Polypropylenfolien unter extrahierenden Bedingungen

Aus den Spritzplatten aus Beispiel 10 werden bei 190 °C jeweils eine 100 µm dicke Preßfolie hergestellt und die auf diese Art erhaltenen Prüfkörper in einem Schnellbewitterungsgerät (®Xenotest 1200) 210 h vorbelichtet. Nach der Vorbelichtung werden die Preßfolien 72 h in refluxierendem Methylenchlorid extrahiert, wobei das Solvens nach jeweils 24 h durch frisches Methylenchlorid ersetzt wird. Anschließend werden die Prüfkörper weitere 146 h in einem Schnellbewitterungsgerät (®Xenotest 1200) belichtet. Als Kriterium der Stabilität der Folie wird die Zunahme des Carbonylindexes herangezogen, der gemäß der Formel CO = E₁₇₂₀/E₂₀₂₀ bestimmt wird. Zu Vergleichszwecken wird eine Folie unter den gleichen Bedingungen, jedoch ohne den Zusatz eines erfindungsgemäßen Stabilisators getestet. Die Versuchsergebnisse sind in Tab. 2 zusammengestellt:

**Tab. 2**

| ΔCO von 100 µm PP-Preßfolien nach insgesamt 356 h Belichtung (210 h Vorbelichtung, Extraktion und 146 h weitere Belichtung, ®Xenotest 1200) | |
|---|---|
| Zusätzlich zur Grundstabilisierung eingesetzter Stabilisator | ΔCO nach insgesamt 356 h Belichtung |
| Keiner | 1,81 |
| Verbindung aus Beispiel 6 | 1,42 |
| Verbindung aus Beispiel 9 | 1,10 |

Ein hoher Wert der CO-Zahl deutet auf eine verstärkte Zersetzung der PP-Matrix hin. Die angeführten Ergebnisse beweisen die stabilisierende Wirkung der erfindungsgemäßen Verbindungen.

### Beispiel 12: Stabilisierende Wirkung in Polycarbonat-Prüfkörpern:

In 500 g Methylenchlorid werden 100 g Polycarbonat-Pulver (®Makrolon 2800) unter Rühren bei Raumtemperatur gelöst. Danach werden 0,2 g des erfindungsgemäßen Stabilisators Zugesetzt und durch Rühren intensiv homogenisiert. Nach Abdampfen des Lösemittels werden Spritzplatten der Dicke 1 mm hergestellt. Diese Prüfkörper werden einer künstlichen Belichtung unterzogen (®Suntest), wobei in regelmäßigen Zeitabständen der Yellowness-Index (YI, Methode ASTM D 1925) gemessen wird.

Eine Zunahme des YI ist hierbei mit der zunehmenden Zersetzung des polymeren Materials gleichzusetzen. Als Vergleich wird ein Polycarbonat-Prüfkörper ohne Zusatz eines Stabilisators mitgetestet. Die Versuchsergebnisse sind in Tab. 3 zusammengefaßt.

**Tabelle 3**

| Yellowness Index (YI) nach 5261 h künstlicher Belichtung im ®Suntest. | |
|---|---|
| Zusätzlich zur Grundstabilisierung eingesetzter Stabilisator | YI nach 5261 h Belichtung |
| Keiner | 55,1 |
| Verbindung aus Beispiel 3 | 45,3 |
| Verbindung aus Beispiel 6 | 45,0 |

Die Ergebnisse zeigen, daß der Einsatz der erfindungsgemäßen Stabilisatoren zu einem verzögerten Polymerabbau führt.

## Patentansprüche

1. Neue Stabilisatoren der allgemeinen Formel (I) 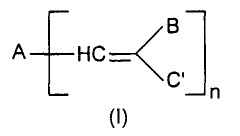 worin
n 1, 2 oder 3 ist und für die Bindungszahl von A steht,
A für n = 1 einen ein- oder mehrfach durch C₁-C₄-Alkyl, Trifluormethyl, C₁-C₄-Alkoxy, Halogen, Cyan, Carboxy, Nitro, Amino, C₁-C₄-Alkylamino, C₁-C₄-Dialkylamino, Acyl oder Acyloxy- substituierten aromatischen oder heteroaromatischen Rest mit 6 bis 20 C-Atomen im Ringgerüst bedeutet, wobei auch eine ein- oder beidseitige Benzanellierung des aromatischen Rests möglich ist,
A für n = 2 einen bivalenten aromatischen Rest mit 6 bis 20 C-Atomen im Ringgerüst bedeutet, der entweder selbst bivalent ist, oder in dem zwei Reste durch eine Brücke verknüpft sind, wobei diese Reste zusätzlich noch ein- oder mehrfach durch C₁-C₄-Alkyl, Trifluormethyl, C₁-C₄-Alkoxy, Halogen, Cyan, Carboxy, Nitro, Amino, C₁-C₄-Alkylamino, C₁-C₄-Dialkylamino, Acyl oder Acyloxy substituiert sein können,
A für n = 3 einen trivalenten aromatischen Rest von 6 bis 20 C-Atomen der Formeln (XIX) bis (XXIII) bedeutet, 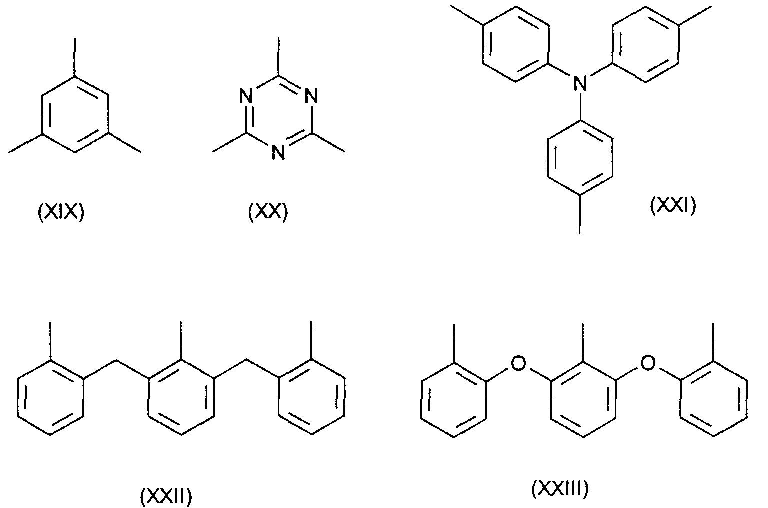 wobei dieser zusätzlich noch ein- oder mehrfach durch C₁-C₄-Alkyl, Trifluormethyl, C₁-C₄-Alkoxy, Halogen, Cyan, Carboxy, Nitro, Amino, C₁-C₄-Alkylamino, C₁-C₄-Dialkylamino, Acyl oder Acyloxy substituiert sein kann,
B -C(=O)-D-E bedeutet
C' Wasserstoff, Cyan oder -C(=O)-D-E bedeutet, wobei
D gleichbedeutend mit -O- oder -NH- ist und
E für D = -O- einen Rest der Formel (XXIV) oder (XXV) bedeutet und 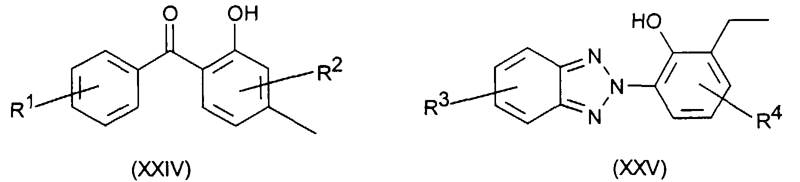
E für D = -NH- ein Rest der Formel (XXV) ist, wobei
R¹, R², R³ und R⁴ einen oder mehrere Substituenten C₁-C₁₈-Alkyl, C₁-C₁₈-Alkoxy, Halogen oder Cyan darstellen.

2. Stabilisatoren gemäß Anspruch 1, dadurch gekennzeichnet, daß
n 1 ist,
A einen ein- oder mehrfach durch C₁-C₂-Alkyl, Trifluormethyl, C₁-C₂-Alkoxy, Halogen, Cyan, Carboxy, Nitro, Amino, C₁-C₂-Alkylamino, C₁-C₂-Dialkylamino, Acyl oder Acyloxy - substituierten aromatischen oder heteroaromatischen Rest mit 6 bis 10 C-Atomen im Ringgerüst bedeutet, wobei auch eine ein- oder beidseitige Benzanellierung des aromatischen Rests möglich ist,
B -C(=O)-D-E bedeutet
C' Wasserstoff, Cyan oder -C(=O)-D-E bedeutet, wobei
D gleichbedeutend mit -O- oder -NH- ist und
E für D = -O- einen Rest der Formel (XXIV) oder (XXV) bedeutet und für D = -NH- ein Rest der Formel (XXV) ist, wobei R¹, R² und R³ C₁-C₁₂-Alkyl, C₁-C₁₂-Alkoxy oder Cyan und R⁴ C₁-C₁₂-Alkyl, C₁-C₁₂-Alkoxy oder Chlor bedeuten.

3. Stabilisatoren gemäß Anspruch 1 und 2, dadurch gekennzeichnet, daß
n 1 ist,
A einen ein- oder mehrfach durch Methyl, Methoxy, Halogen, Cyan, Nitro, Dimethylamino, substituierten aromatischen Rest mit 6 bis 8 C-Atomen bedeutet,
B -C(=O)-D-E bedeutet
C' Wasserstoff oder -C(=O)-D-E bedeutet, wobei
D gleichbedeutend mit -O- oder -NH- ist und
E für D = -O- einen Rest der Formel (XXIV) oder (XXV) bedeutet und für D = -NH- ein Rest der Formel (XXV) ist, wobei R¹ bis R⁴ unabhängig voneinander einen oder mehrere Methylreste darstellen.

4. Verfahren zur Herstellung der Verbindungen (I), dadurch gekennzeichnet, daß Verbindungen der allgemeinen Formeln (XXVI) oder (XXVII), 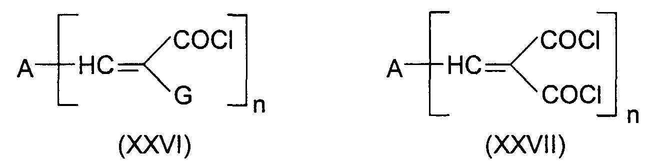 wobei G dann die Bedeutung von Wasserstoff oder Cyan hat, mit Verbindungen der allgemeinen Formel (XXVIII)
E―D―H (XXVIII)
wobei D und E die obigen Bedeutungen besitzen und es sich im Falle von D = -NH- auch um das Hydrochlorid der eingesetzten Verbindung handeln kann, umgesetzt werden.

5. Verfahren zur Herstellung der Verbindungen (I), dadurch gekennzeichnet, daß Verbindungen der allgemeinen Formel (XXIX)
A⁅CHO]ₙ (XXIX)
mit Verbindungen der allgemeinen Formeln (XXX) oder (XXXI), 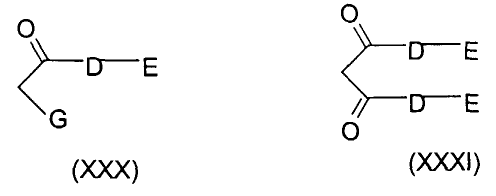 wobei D, E und G die obigen Bedeutungen besitzen, umgesetzt werden.

6. Verwendung der Verbindungen der Formel (I) zum Stabilisieren von organischen Material, insbesondere als Lichtschutzmittel für Kunststoffe, Anstrichmittel, Lacke und Öle.

7. Verfahren zum Stabilisieren von polymeren Material gegen den zerstörerischen Abbau durch Licht, Sauerstoff und Wärme, dadurch gekennzeichnet, daß man dem zu stabilisierenden Material eine Verbindung der Formel (I) nach Anspruch 1 bis 3 in einer Konzentration von 0,001 bis 5, vorzugsweise von 0,02 bis 1 Gew.-%, bezogen auf das organische Material, vor, während oder nach seiner Herstellung zusetzt.

8. Stabilisiertes organisches Material, welches mindestens eine der oben genannten Verbindungen der Formel (I) enthält.
